Europäisches Patentamt

European Patent Office

Office européen des brevets

(11) Publication number: 0 339 695

A2

# EUROPEAN PATENT APPLICATION

(21) Application number: 89200145.4

(51) Int. Cl.4: A61K 47/00

(22) Date of filing: 23.01.89

(30) Priority: 29.04.88 US 188310

(43) Date of publication of application:
02.11.89 Bulletin 89/44

(84) Designated Contracting States:
AT BE CH DE ES FR GB GR IT LI LU NL SE

(71) Applicant: STICHTING VOOR DE TECHNISCHE
WETENSCHAPPEN
Van Vollenhovenlaan 661
NL-3527 JP Utrecht(NL)

(72) Inventor: Bloemhoff, Willem
Rijndijk 280
2394 AP Hazerswoude(NL)
Inventor: Drijfhout, Jan Wouter
Westduinweg 161
2583 AA The Hague(NL)

(74) Representative: de Bruijn, Leendert C. et al
Nederlandsch Octrooibureau
Scheveningseweg 82 P.O. Box 29720
NL-2502 LS 's-Gravenhage(NL)

(54) A process for preparing an antigenic or immunogenic conjugate as well as the use of such conjugates.

(57) The invention relates to a process for preparing an antigenic or immunogenic conjugate such as a multiple antigenic peptide system. A branched structure based on amino acids such as lysine is reacted with an antigenic or immunogenic compound. Preferably said compound is coupled to a residue containing the 3-nitro-2-pyridine sulfenyl group. The conjugates obtainable according to this process are used for the preparation of pharmaceutical products.

fig-1

## A process for preparing an antigenic or immunogenic conjugate as well as the use of such conjugates.

The invention relates to a process for preparing an antigenic or immunogenic conjugate such as a multiple antigenic peptide system.

For the description of the present invention the following abbreviations are used: Boc, tert-butyloxycarbonyl; BSA, bovine serum albumin; DCC, N,N'-dicyclohexylcarbodiimide; DIPEA, N,N-diisopropyl ethylamine; DMA, N,N-dimethylacetamide; Fmoc, 9-fluorenylmethyloxycarbonyl; DMAP, 4-(dimethylamino)-pyridine; DTT, dithiothreitol; GPC, gel-permeation chromatography; HBTU, O-benzotriazolyl-N,N,N',N'-tetramethyluronium hexafluorophosphate; HOAc, acetic acid; HOBt, 1-hydroxybenzotriazole; i-PrOH, 2-propanol; MAP, multiple antigenic peptide; Nle, norleucine; Npys, 3-nitro-2-pyridinesulfenyl; Pfp, pentafluorophenyl; RP-HPLC, reversed phase high-performance liquid chromatography; TFA, trifluoroacetic acid.

A multiple antigenic peptide, a so-called MAP, is known from D.N. Posnett, H. McGrath and J.P. Tam, J. Biol. Chem., 263, 1719 (1988) and J.P. Tam. Proc. Natl. Acad. Sci. USA., 85, 5409 (1988). In fact said MAP is a conjugate based on a branched lysine structure and, in short, its preparation is as follows. To a resin containing a reference amino acid, Boc-Lys(Boc)-OH was coupled. The product obtained was deprotected with TFA, thus affording a dipeptide in which the and $\alpha$- and $\epsilon$-amino group of the lysine residue are free. By performing two additional synthetic cycles, in which Boc-Lys(Boc)-OH was used as the incoming amino acid, a branched octapeptide, having eight amino groups, was produced. To this branched structure eight epitope sequences were attached by stepwise peptide synthesis.

During the stepwise peptide synthesis referred to in the above undesired side reactions may occur. On the other hand the known synthesis may be troublesome due to steric hindrance on the polymer. In addition, according to the known method the branched structure has to be prepared every time a coupling is derived. This method is only suited for the coupling of peptide antigens. E.g. polysaccharide antigens cannot be prepared in this way.

According to the present invention a favourable preparation of a MAP is provided. Surprisingly, it was found that the branched amino acid structure such as the lysine structure referred to in the above as well as the peptide can be prepared separately and subsequently coupled.

The invention relates to a process for preparing an antigenic or immunogenic conjugate such as a multiple antigenic peptide system comprising reaction of a branched structure based on amino acids with an antigenic compound such as an immunogenic peptide.

In the process according to the invention the branched structure contains preferably a group of the type as depicted in formula 4 or 5, wherein A is a group derived from an amino acid containing at least one COOH group and two NH2 groups such as lysine and/or ornithine, and B is a group derived from an amino compound such as a diamino compound or an amino acid.

Preferably A is a group derived from an amino acid which is non-toxic for mammals, e.g. lysine.

B may be derived from an amino compound which preferably differs from amino acids in the other parts of the conjugates to be prepared. It appeared that 4-aminobutyric acid is well suited.

In the branched structure having the formula 4 or 5 the end groups A are preferably coupled to a solubilizing group C, such as a group derived from polar amino acids. Such a structure is depicted in formula 6. It appeared that glutamic acid is well suited. Such a group C makes the conjugate well soluble in water. It is also possible to insert a spacer group (e.g. by coupling additional amino acid residues) between groups A and C in order to modify the "bulkyness" of the conjugate to be prepared. By the use of such known spacers the steric hinderence in the molecule may be controlled.

In the structure according to formulae 4 and 5 there are 4 and 8 end amino groups respectively. It is possible, however, that more amino acids having two amino groups are coupled so that e.g. 16 or 32 end amino groups are present. Probably the maximum of amino acids will depend on steric hindrance.

In practice the end groups A or C are functionalized in such a way that they contain thiol groups or temporarily protected thiol groups. Such protected thiol groups may be represented by SC-CO-X, X being alkyl, aryl or aralkyl, e.g. S-acetyl. Preferably, the peptide used in the process according to the invention has been coupled to a residue containing the 3-nitro-2-pyridine sulfenyl group, e.g. the S(3-nitro-2-pyridinesulfenyl)cysteinyl group.

According to the method of the invention various protein fragments may be coupled to the branched structure, e.g. 9-21 sequence of glycoprotein D of HSV-1 (9-21). An average expert in this field will be able to choose all other possible protein fragments.

The invention also relates to an antigen or an immunogen on the basis of a conjugate prepared according to the above process, e.g. an immunogen which is active against HIV or herpes simplex viruses (HSV).

In general, the present invention also relates to the use of conjugates obtainable according to the process of the invention for the preparation of pharmaceutical products such as vaccins, antigens, immunogens or specific kits.

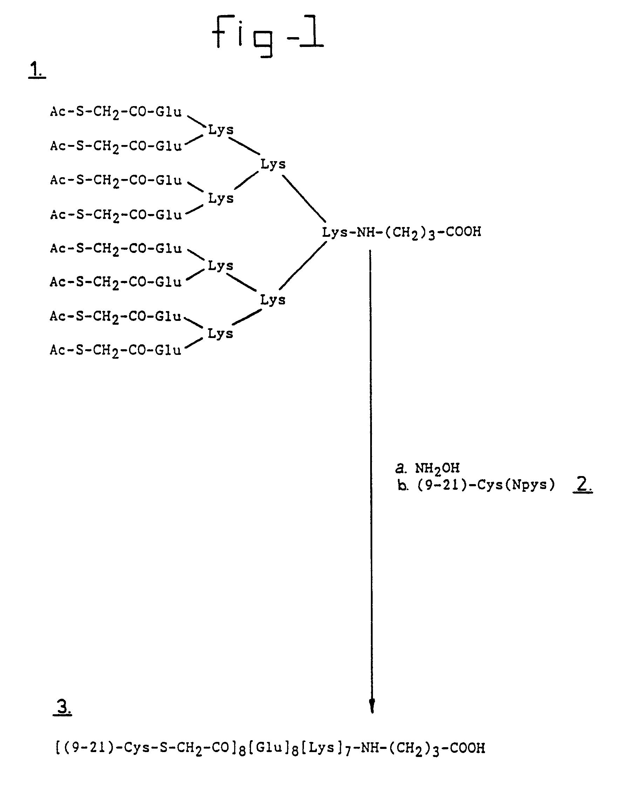

The invention is illustrated by means of a typical preparation shown in the reaction scheme according to Figure 1.

According to said reaction scheme a MAP containing the 9-21 sequence of glycoprotein D of HSV-1 (9-21) is prepared. The branched lysine structure having the formula 1 is prepared as follows. N-Fmoc-4-aminobutyric acid is coupled to a Wang-resin (see J. Chem. Soc. Perkin Trans. (1986), 125-137). Three synthetic cycles are performed, using Fmoc- Lys(Fmoc)-OPfp as the incoming amino acid derivative. To each of the eight amino groups of the branched octapeptide thus obtained, glutamic acid and S-acetyl mercaptoacetic acid are coupled, respectively. After cleavage of the resin and removal of the t-butyl protecting groups of the Glu-residues with aqueous TFA, branched structure having the formula 1 is obtained. It contains eight acetyl protected thiol functions, and it is water soluble due to the presence of eight Glu-residues. To this branched structure (9-21)-Cys(Npys) (formula 2), which was synthesized independently, is coupled. The MAP (formula 3) obtained is purified by GPC. This branched protein, with a molecular weight of over 15,000, contains 78% immunogen by weight. To prove the presence of the peptide in this compound, the MAP is reduced with DTT and the reaction mixture analyzed by RP-HPLC. Two products can be detected in the reaction mixture. One of the products coeluted with (9-21)-Cys, and the other with the product which is formed when the S-acetyl protected branched structure (formula 1) is treated with hydroxylamine.

Of course, generally known methods for coupling peptides to proteins may be used for the coupling of the peptide to the branched amino acid structure. In principle all known methods for preparing a peptide-protein conjugate may be used.

A known method to connect amino groups in a peptide with those in a protein is to make use of glutaric dialdehyde (glutaraldehyde). Unfortunately, this procedure is not reproducible and yields a large number of products, presumably due to the fact that the peptide reacts either via its N-terminus or via a lysine side chain. Also, protein-protein and peptide-peptide coupling products are formed.

Other known methods of conjugation include amide-formation using carbodiimide activation or water-soluble active esters of peptides and coupling via bis-diazotized benzidine. However, these methods have the same drawbacks as the method with glutaric dialdehyde.

A more selective method of coupling the peptide to the protein is based on the reaction between a thiolated peptide and a protein which has been functionalized with 3-maleimidobenzoic acid N-succinimidyl ester (MBS) or N-succinimidyl 3-(2-pyridyldithio)propionate (SPDP). However, the thiolated peptides are susceptible to (air)oxidation.

The above mentioned Npys method may be used also for other conjugation methods for coupling of peptides and proteins. It appeared that this method is selective and mild and is easily carried out yielding well-defined conjugates.

Accordingly, the present invention also relates to a process comprising the following steps:

a) elongation of an antigen, e.g. a peptide or a saccharide with a residue containing the 3-nitro-2-pyridinesulfenyl group;

b) reaction of the product of step (a) with a functionalized protein to form said peptide-protein conjugate in which the said product is attached to the protein by a disulfide bond.

Preferably the reaction or reactions in step (a) leading to said elongation are performed while the peptide is anchored to a solid support. In general, the peptide is synthesized by the method of solid phase peptide synthesis, as described in detail in Science Tools, Vol. 33, No. 1, (1986), pages 9-16 and Chemica Scripta (1985), 25, pages 121-131.

According to the invention the elongation of step (a) preferably leads to a product containing the S(3-nitro-2-pyridinesulfenyl)cysteinyl group. In particular the residue mentioned in step (a) is identical with the S(3-nitro-2-pyridinesulfenyl)cysteinyl group. It may be desirable to insert a spacer between Npys-cysteine and the peptide, e.g. by coupling with Boc-Cys(Npys)-NH-$(CH_2)_n$-COOH. It is also convenient to couple amino acid residues to form a spacer.

The 3-nitro-2-pyridine sulfenyl compound used for elongation in step (a) is preferably an N-protected-S-(Npys)-cysteine derivative, e.g. Boc-Cys(Npys)-OH or Boc-Cys(Npys)-Opfp. The preparation of Boc-Cys-(Npys)-OH and the use of the S(Npys) group for unsymmetrical disulfide bond formation is known from Int. J. Peptide Protein Res. 28, (1986), pages 107-112. In this reference it is also suggested to crosslink an enzyme to a site-directing antibody via a peptide or other compound that contains the S-Npys moiety. However, from this reference it can be concluded only that the Npys group is suitable in fragment coupling of peptides. According to the present invention the Npys group serves as a stable sulphur protecting group during peptide synthesis, cleavage and purification procedures, whereas the same function activates the peptide for disulfide formation in the conjugation stage.

In the process of the invention it is preferred to use a protein which has been functionalized in such a way that it contains thiol groups or temporarily protected thiol groups. Such protected thiol groups are in particular equal to the group S-CO-X, X being an alkyl, an aryl or an arylalkyl group, e.g. the S-acetyl group.

The method of the invention is further illustrated by means of the reaction scheme according to Fig. 3.

According to the embodiment of Fig. 3 step (a) is started with a protected peptide covalently linked to a resin (P).

After N-terminal deprotection Boc-Cys(Npys)-0H is coupled to the peptide chain using e.g. HBTU/DIPEA and the S(Npys)-cysteinyl-peptide is deprotected and cleaved from the resin (preferably with TFA) and purified. Identification of Npys-peptide in HPLC-analysis is easy because the Npys group serves as a "chromatophoric handle".

The carrier-protein (e.g. BSA) is functionalized with e.g. S-acetylmercaptosuccinic anhydride, at a pH of about 8.0, preferably in an N-ethylmorpholine buffer.

Although the reaction between a thiol and an S-Npys compound can be performed over a wide pH range, the reaction is slow at low pH. The reaction proceeds smoothly under neutral or slightly basic conditions. The acetyl protection is removed in situ by adding $NH_2OH.HCl$ to a solution of S-acetyl functionalized BSA and Npys- cysteinyl-peptide in a buffer at pH 7-8, upon which the conjugation reaction starts and is completed within a few hours. In this manner the thiol remains protected as long as possible to prevent undesirable (air)oxidation. Due to liberation of 3-nitro-2-thiopyridone, which is the driving force for the conjugation reaction, the colour of the solution changes from light to dark yellow.

The reaction mixture is separated by gel filtration into a protein fraction, a peptide fraction (in case of the use of an excess Npys-cysteinylpeptide) and a third, yellow fraction containing the 3-nitro-2-thiopyridone. The incorporation of peptide as measured by the amount of 3-nitro-2-thiopyridone liberated, proved to be in the range of 11-15 mol peptide per mol protein. This is confirmed by amino acid analyses of the conjugates.

If an aliquot of the conjugate is reduced using tri-n-butylphosphine, the liberated peptide has the same chromatographic behaviour as the peptide obtained on reducing the Npys-peptide, strongly indicating that the peptide was not modified during the conjugation procedures.

The benefits of the conjugation method according to the invention may be summarized as follows:

**a.** The S-protected peptide is not susceptible to undesired disulfide formation;

**b.** The peptide can be conjugated to the protein in a facile and unambiguous way;

**c.** The amount of incorporation can be easily controlled;

**d.** The amount of incorporation can be easily determined; and

**e.** The Npys chromophore allows flexible detection of the peptide.

The process according to the invention may be carried out advantageously with the use of a kit comprising e.g. the following components:

A1 : the 3-nitro-2-pyridine sulphenyl compound, e.g. Boc-Cys(Npys)-Opfp; A2 : a catalyst such as HOBt;

B1 : functionalized carrier protein, e.g. BSA functionalized by reaction with S-acetylmercaptosuccinic anhydride;

B2 : buffer in which the conjugation reaction is to be carried out;

B3 : hydroxylamine.HCl.

With A1 and A2 e.g. Boc-Cys(Npys) may be coupled to the solid-phase peptide (having a free N-terminal group).

After deprotection and cleavage from the polymer (and optionally purification) the peptide may be conjugated with the carrier protein. For that purpose the S-Npys containing peptide is dissolved in a part of B2. B1 is dissolved in another part of B2. Both parts are mixed. After adding B3 to this mixture and stirring for some time the conjugation is completed. Then the peptide-protein conjugate may be purified by dialysis and/or gel-filtration chromatography.

The peptide-protein conjugates prepared according to the method of the invention may be used advantageously for immunization, i.e. vaccination. In principle all types of antigenic peptides, i.e. potentially immunogenic peptides, may be coupled to a suitable carrier protein according to the method of the invention.

Accordingly, the invention relates to vaccines on the basis of a peptide-protein conjugate prepared according to the method of the invention. For instance, the invention may relate to a vaccine being active against herpes simplex viruses (HSV).

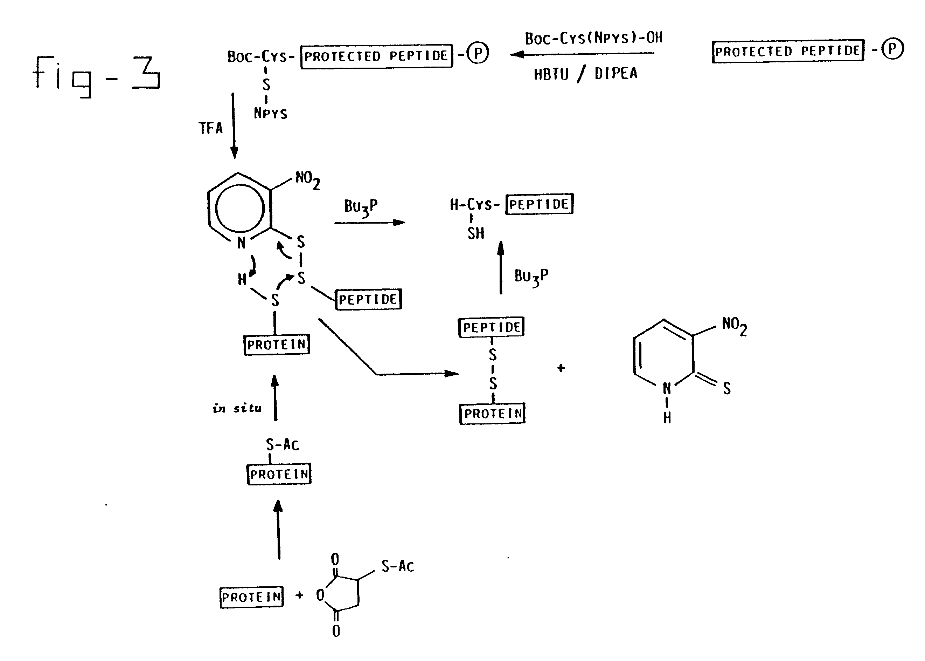

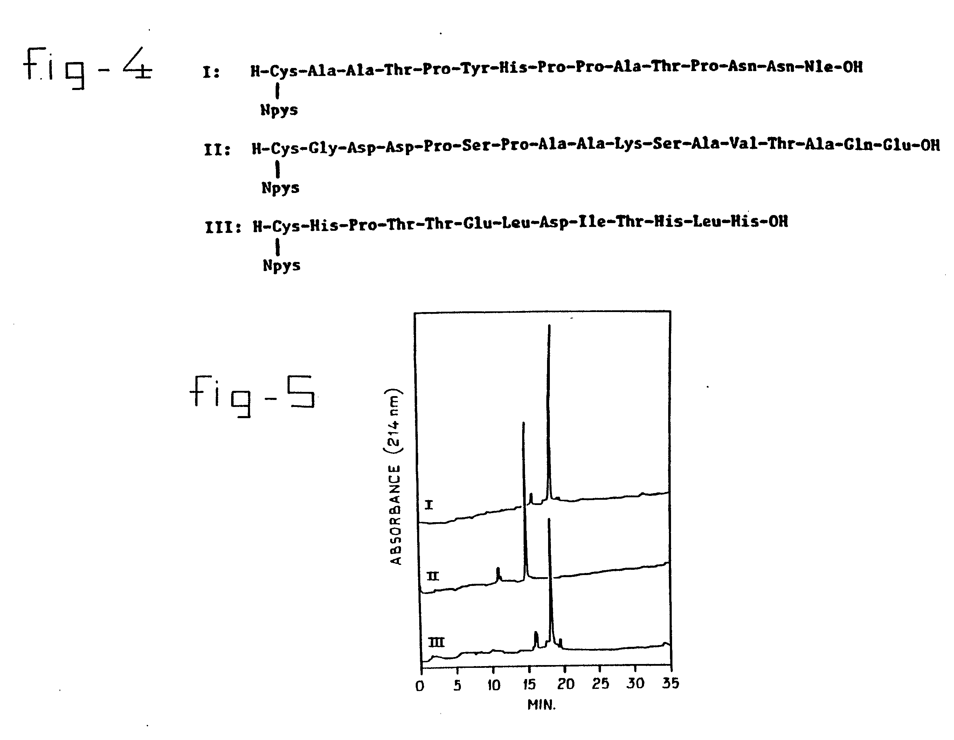

Examples of HSV-peptides which may be conjugated according to the method of the invention, are depicted in Figure 4. These peptides may be prepared according to the method described in J. Chem. Soc. Perkin Trans. (1986), I, pages 125-137.

The present invention will now be further illustrated by the following examples.

## Example 1

Preparation of (9-21)-Cys(Npys) (formula 2)

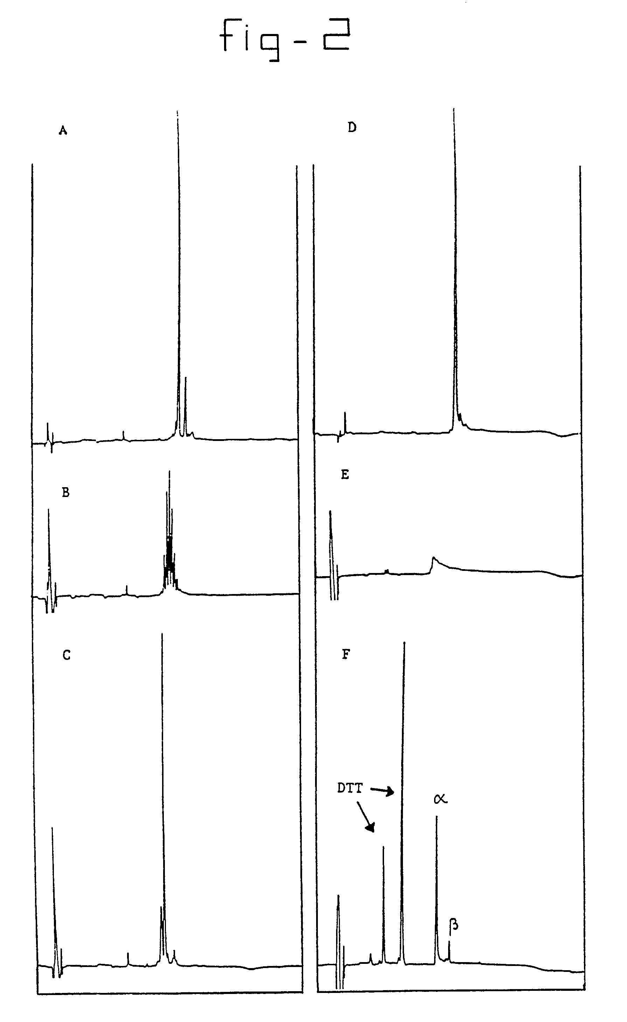

Fmoc-Cys(tBu)-OH was converted to its symmetrical anhydride by treatment with DCC according to standard procedures described by Dryland and Sheppard in J. Chem. Soc. Perkin. Trans I (1986), 125-137. To a solution of 0.5 mmol of this symmetrical anhydride in 4 ml of DMA, 800mg Wang resin (85 $\mu$mol/g) and 24 mg (0.2 mmol) DMAP were added. After gentle shaking for 2 h (room temperature) the resin was collected by filtration and washed with DMA. Subsequently HSV-gD-10-24 was synthesized. After the last Fmoc-deprotection the peptide chain was elongated by coupling with the symmetrical anhydride of Boc-Leu-OH. The resin was washed with DMA and treated for 90 min with Npys-Cl (freshly prepared from 93 mg (0.3 mmol) 3,3'-dinitro-2,2'-dipyridyldisulfide. The resin was washed with DMA and MeOH, and dried in vacuo over silica drying gel. An aliquot (400 mg) was treated with 5 ml TFA containing 5% thioanisole as a scavenger for 8 h at room temperature. The resin was removed by filtration and washed with 15 ml TFA. The combined filtrates were evaporated to dryness in vacuo and the residue was triturated with ether (3 x 20 ml), affording crude peptide having the formula 2 as a yellow powder. The peptide was desalted over a Sephadex G-25 column (150 x 1.0 cm; 0.05 M HOAc), lyophilized and analyzed by RP-HPLC (see Fig. 2D). Amino analysis: Asp 2.92 (3), Gly 0.97 (1), Ala 1.07 (1), Cys 0.63 (1), Leu 0.92 (1), Nle 1.00 (1), Phe 0.86 (1), Lys 1.83 (2), Arg 1.81 (2). This peptide was conjugated to a branched structure as described in Example 2.

## Example 2

Preparation of a MAP containing 9-21 (formula 3).

The branched oligo-lysine construct having the formula 1 was synthesized as follows. A Wang resin (500 mg, 46 $\mu$mol) was treated with 1). the symmetrical anhydride of Fmoc-4-aminobutyric acid (0.5 mmol) and DMAP (24 mg, 0.2 mmol) in 3 ml DMA for 2 h. After removal of the Fmoc-protection (46 $\mu$mol) with 20% piperi dine in DMA, coupling was performed as follows.

| | | | | |
|---|---|---|---|---|
| 2). | Fmoc-Lys(Fmoc)-OPfp | 150 mg, | 0.2 mmol | |
| | HOBt | 27 mg, | 0.2 mmol | loading 94 $\mu$mol |
| 3). | Fmoc-Lys(Fmoc)-OPfp | 225 mg, | 0.3 mmol | |
| | HOBt | 40 mg, | 0.3 mmol | loading 184 $\mu$mol |
| 4). | Fmoc-Lys(Fmoc)-OPfp | 310 mg, | 0.4 mmol | |
| | HOBt | 53 mg, | 0.4 mmol | loading 360 $\mu$mol |
| 5). | Fmoc-Glu(tBu)-OPfp | 591 mg, | 1.0 mmol | |
| | HOBt | 130 mg, | 1.0 mmol | loading 356 $\mu$mol |
| 6). | Ac-S-CH$_2$CO-OPfp | 300 mg, | 1.0 mmol | |
| | HOBt | 130 mg, | 1.0 mmol | loading n.d. |

After washing the resin with DMA and MeOH, it was dried in vacuo; yield 640 mg peptide-resin. A part (350 mg) was treated with 5 ml 95% aqueous TFA for 90 min. After removal of the resin by filtration, the filtrate was evaporated to dryness in vacuo. The residue was triturated with ether (3 x 20 ml) and dried in vacuo over silica drying gel. The S-acetyl protected branched structure having the formula 1 obtained was used without further purification.

To a solution of 1.0 μmol of branched structure having the formula 1 (8 μmol S-Ac) in 2.0 ml Na-phosphate buffer (0.2 M, pH = 6.5) was added hydroxylamine.HCl (7.0 mg, 100 μmol). The solution was stirred for 4 h in a nitrogen atmosphere. A solution of 16 μmol (9-21)-Cys(Npys) having the formula 2 in 1.6 ml Na-phosphate buffer (0.2 M, pH = 6.5) was added and stirred was continued for 4 h. The reaction mixture was applied to a Sephadex G-25 column (150 × 1.0 cm). Elution with 0.05 M HOAc afforded the MAP having the formula 3.

Amino acid analysis: Asp 24.7 (24), Glu 7.1 (8), Pro n.d., Gly 9.8 (8), Ala 8.3 (8), Cys n.d., Leu 7.9 (8), Nle 7.6 (8), Phe 7.9 (8), Lys 22.8 (23), Arg 14.7 (16), 4-aminobutyric acid n.d.

Fig. 2A-E show the results of RP-HPLC analysis of the materials involved in the preparation of the MAP.

Fig. 2A: structure having the formula 1.
Fig. 2B: structure having the formula 1 with NH$_2$OH after 2 h.
Fig. 2C: structure having the formula 1 with NA$_2$OH after 4 h.
Fig. 2D: peptide having the formula 2.
Fig. 2I: MAP having the formula 3.

An aliquot of the MAP (about 0.1 μmol), dissolved in a Na-phosphate buffer (0.1 M, pH = 7), was treated with an excess dithiothreitol (DTT) for 1 h. RP-HPLC analysis of the reaction mixture (see Fig. 2F) showed two peptide peaks. The peaks α and β represent (9-21)-Cys and (HS-CH$_2$-CO-Glu)$_8$(Lys)$_7$-NH-(CH$_2$)3-COOH, respectively as indicated by coelution experiments.

## Example 3

This example describes the preparation of various starting materials.

### Fmoc-Lys(Fmoc)-0H

To a solution of lysine.HCl (0.91 g, 5 mmol) in 40 ml water, was added NaHCO$_3$ (1.68 g, 20 mmol) and Na$_2$CO$_3$ (1.06 g, 10 mmol). The stirred mixture was cooled in ice and a solution of Fmoc-ONSu (3.37 g, 10 mmol) in 40 ml dioxane was added dropwise (1 h). Stirring was continued for 4 h at room temperature. The clear solution was then poured into 150 ml ice water containing 4 ml of concentrated HCl. The precipitate obtained was collected by filtration, washed with water (50 ml) and dissolved in CH$_2$Cl$_2$ (100 ml). The solution was washed with 0.1 M KHSO$_4$ (2 × 50 ml), dried (MgSO$_4$), filtered and evaporated to dryness in vacuo. The solid residue was recrystallized from methanol, affording 1.70 g (= 57%) Fmoc-Lys(Fmoc)-OH. m.p. = 118-120°C.

$^{13}$C-NMR (DMSO-d6): δ 23.0 (CH$_2$); 29.0 (CH$_2$); 30.5 (CH$_2$); 39.9 (CH$_2$); 46.7 (CH, Fmoc); 46.9 (CH, Fmoc); 53.9 (Cα); 65.3 (CH$_2$, Fmoc); 65.7 (CH$_2$, Fmoc); 120.1 (4 × CH, arom.); 125.2 (4 × CH, arom.); 127.0 (4 × CH, arom.); 127.6 (4 × CH, arom.); 140.7 (4 × C, arom.); 143.8 (2 × C, arom.); 144.0 (2 × C, arom.); 156.1 (C = 0, Fmoc); 156.2 (C = 0, Fmoc); 174.1 (C = 0, acid).

### Fmoc-Lys(Fmoc)-OPfp

To a stirred solution of Fmoc-Lys(Fmoc)-0H (1.18 g, 2 mmol) and pentafluorophenol (368 mg, 2 mmol) in 40 ml acetonitrile was added N,N′-dicyclohexylcarbodiimide (412 mg, 2 mmol). Stirring was continued for 3 h. 100 ml CH$_2$Cl$_2$ was added, the solution was filtered and the filtrate was evaporated to dryness in vacuo. The oil, dissolved in 20 ml methanol/CH$_2$Cl$_2$ (1/19), was applied to a silica gel column (15 g). The effluent obtained on elution with CH$_2$Cl$_2$ (60 ml) was evaporated to dryness in vacuo. The residue was crystallized from 120 ml CH$_2$Cl$_2$/ether (1/5). Yield: 1.17 g (= 77%). m.p. = 171-173°C (dec.).

$^{13}$C-NMR (DMSO-d6): δ 22.5 (CH$_2$); 28.8 (CH$_2$); 29.9 (CH$_2$); 39.7 (CH$_2$); 46.6 (CH, Fmoc); 46.8 (CH, Fmoc); 53.8 (Cα); 65.2 (CH$_2$, Fmoc); 65.8 (CH$_2$, Fmoc); 120.1 (4 × CH, arom.); 124.7 (m, C, OPfp-arom.); 125.1 (4 × CH, arom.); 127.0 (4 × CH, arom.); 127.6 (4 × CH, arom.); 134.9-143.7 (m, 5 × C-F); 140.7 (4 × C, arom.); 143.6 (2 × C, arom.); 143.8 (2 × C, arom.); 156.1 (C = 0, Fmoc); 156.2 (C = 0, Fmoc); 169.1 (C = 0, ester).

S-Acetyl mercaptoacetic acid

Acetylchloride (15.8 g, 0.2 mol) was added dropwise to mercaptoacetic acid (18.4 g, 0.2 mol) in 15 min. The reaction mixture was heated for 15 min. at 70° C and subsequently distilled in vacuo yielding 10.0 g (=37%) S-acetyl mercaptoacetic acid as a light yellow oil. b.p. = 101-102° C (1 mm Hg). Lit[10]: b.p. = 158-159° C (17 mm Hg).
$^1$H-NMR (CDCl$_3$): $\delta$ 2.42 (s, 3H); 3.75 (s, 2H); 10.78 (br, 1H, OH)

S-Acetylmercaptoacetic acid pentafluorophenyl ester

To a solution of S-acetylmercaptoacetic acid (2.68 g, 20 mmol) and pentafluorophenol (3.75 g, 20.4 mmol) in 20 ml CH$_2$Cl$_2$ was added dropwise a solution of N,N$^{'}$-dicyclohexylcarbodiimide (4.50 g, 21,8 mmol) in 40 ml CH$_2$Cl$_2$. After stirring for 17 h the reaction mixture was filtered, and evaporated to dryness in vacuo. The residue obtained was dissolved in 25 ml CH$_2$Cl$_2$ and applied to a silica gel column (25 g). The effluent obtained on elution with CH$_2$Cl$_2$ (100 ml) was evaporated to dryness in vacuo. The residue was crystallized from pentane (80 ml).
Yield: 4.51 g (=75%). m.p. = 40-41° C.
$^1$H-NMR (CDCl$_3$): $\delta$ 2.45 (s, 3H); 4.02 (s, 2H).
$^{13}$C-NMR (CDCl$_3$): $\delta$ 29.5 (CH$_3$); 30.3 (CH$_2$); 124.7 (C-1, m, arom.); 134.9-143.7 (5 x C-F, m); 165.0 (C=0, ester); 192.7 (C=0, acetyl).

N-Fmoc-4-aminobutyric acid

To a stirred solution of NaHCO$_3$ (840 mg, 10 mmol) and Na$_2$CO$_3$ (530 mg, 5 mmol) in 40 ml water, was added 4-aminobutyric acid (515 mg, 5 mmol). The mixture was stirred until a clear solution was obtained (15 min.) and a solution of Fmoc-ONsu (1.69 g, 5 mmol) in 40 ml dioxane was added dropwise (1 h). Stirring was continued for 4 h and the reaction mixture was poured into 150 ml ice water containing 3 ml of concentrated HCl. The suspension obtained was extracted with CH$_2$Cl$_2$ (3 x 50 ml), the combined organic layers were dried (MgSO$_4$), filtered and evaporated to dryness in vacuo. The residue was crystallized from methanol (35 ml), affording 1.40 g (=86%) Fmoc-4-aminobutyric acid as white crystals. m.p. = 166-168° C.
$^{13}$C-NMR (CDCl$_3$): $\delta$ 24.9 (CH$_2$); 30.9 (CH$_2$); 39.7 (CH$_2$); 46.9 (CH, Fmoc); 65.3 (CH$_2$), Fmoc); 120.1 (CH, arom.); 125.1 (CH, arom.); 127.0 (CH, arom.); 127.6 (CH, arom.); 140.8 (C, arom.); 144.0 (C, arom.); 156.2 (C=0, Fmoc); 174.3 (C=0, acid).

Example 4

Materials and Methods.

Reagents and solvents were used without further purification, except for the following:
DMA was distilled in vacuo under nitrogen from 2,4-dinitrofluorobenzene before use; ether was distilled from P$_2$O$_5$, piperidine from KOH, dioxane and ethylenediamine from CaH$_2$ and N-ethylmorpholine from NaOH. HOBt was dried in vacuo at 50° C over P$_2$O$_5$ for 72 h.
HPLC grade acetonitrile was purchased from Rathburn, Walkerburn, Scotland. Sephadex 6-25 (superfine) and G-50 (fine) were obtained from Pharmacia Fine Chemicals, Uppsala, Sweden. Fmoc-L-amino acid pentafluorophenyl esters were either purchased from Cambridge Research Biochemicals Ltd, Cambridge, England, or previously prepared according to Synthesis (1983), pages 325-327. Fmoc-Asn-4-nitrophenyl ester was synthesized according to J. Chem. Soc. Perkin Trans. (1981), l, pages 538-546. S-acetylmercaptosuccinic anhydride was obtained from Aldrich. BSA (fraction V, lot 12F-0649) was purchased from Sigma. Pepsyn K, a physically supported acrylamide polymer was used as a resin (0.1 mequiv./g, lots 1338/131, 11065/402, 1254/549) and purchased from Cambridge Research Biochemicals Ltd, Cambridge, England. Boc-Cys(Npys)-OH was synthesized according to Chem. Lett., (1982), pages 921-924, starting from 2-chloro-3-nitropyridine (Janssen) and Boc-Cys(Trt)-OH (Sigma). HBTU was synthesized according to Tetrahedron, (1978), pages 1269-1272. (4-Hydroxymethylphenoxy)acetic acid N-succinimidyl ester was prepared from (4-hydroxymethylphenoxy)acetic acid (see Int. J. Peptide Protein Res. (1982), 20, pages 451-454), N-hydroxysuccinimide and DCC. Peptide synthesis was performed using a semi-automatic flow-reactor, containing the following parts: several three-way valves mod.802-2312-1-0 (Mace, South El Monte, California, USA), a Gilson-302 HPLC pump, a Cecil Instruments CE-212 spectrophotometer equipped with a 1 mm flowcell (2120773), a Kipp & Zonen BD 41 recorder and a glass reaction column (100 x 10 mm, Omnifit Ltd, Cambridge, England), parts connected by way of PTFE tubing (0.7 x 1.6 mm). The pump and the valves were computer controlled. For HPLC a column (250 x 4.0 mm) packed with Lichrocart 5C$_{18}$ 5$\mu$m (E.Merck, Darmstadt, GFR) was used; mobile phase: 0.1% TFA in water (liquid A), 0.1% TFA in water/acetonitrile 1/2 (v/v) (liquid B); linear gradient: 10-100% B over 30 min; flow rate 1.0 ml/min; detection at 214 or 329 nm. Peptides were hydrolyzed for amino acid analysis with constant boiling (5.7 N) HCl in vacuo at 110°C in sealed glass tubes for 20-24 h. The amino acid content of the hydrolysates was determined with a Kontron Liquimat III analyzer. For optical density measurements a Varian Cary 219 spectrophotometer was used.

## Experimental Procedures

All experiments were performed at room temperature unless stated otherwise.

## Synthesis of S(Npys)-cysteinylpeptides

Three HSV-peptides were synthesized, designated as [Nle$^{315}$]-HSV-1-gD-302/315, HSV-1-gC-402/417 and HSV-1-gH-204/215. Amino acids in peptides are numbered according to the numbering of the published amino acid sequences of mature glycoproteins gD (Science 218, (1982), pages 381-384), immature glycoprotein gC (J. Virol, 45, (1983), pages 634-647) and immature glycoprotein gH (Nucleic Acids Res. 14, (1986), pages 4281-4292) of HSV type 1.

For peptide synthesis the stepwise semi-automatic solid-phase method according to the general procedures of Atherton & Sheppard was used (J. Chem. Soc. Perkin Trans. (1986), I, pages 125-137; J. Chem. Soc. Chem. Commun. (1985), pages 165-166). Prior to peptide synthesis pepsyn K was functionalized by reaction with ethylenediamine, Fmoc-Nle-OPfp, piperidine and (4-hydroxymethylphenoxy)-acetic acid N-succinimidyl ester, respectively. The first residue was attached to the resin using 6 equiv. Fmoc-amino acid active ester and 0.6 equiv. 4-dimethylaminopyridine over a period of 40 min. The following acylation reactions were performed by using equal amounts of Fmoc-amino acid pentafluorophenyl esters and HOBt (4 equiv.), except in the case of Asn, which was coupled through the 4-nitrophenyl ester and HOBt. Coupling times were 1 h. For all reactions and washings DMA was used as a solvent. Deprotection reactions were carried out in 20% piperidine in DMA (8 min). Completeness of couplings was checked with the Kaiser ninhydrin test (Anal. Biochem. 34, (1970), pages 595-598). The course of synthetic cycles was followed by monitoring the column effluent at 312 nm. After coupling and deprotection of the N-terminal residue of the peptide Boc-Cys(Npys)-OH (10 equiv.) was coupled using HBTU (10 equiv.) and DIPEA (10 equiv.) in DMA for 1.5 h (Synthesis, (1984), pages 572-574). The resin was washed successively with DMA, HOAc in DMA, DMA, i-PrOH, MeOH and ether, and dried in vacuo over silica blue.

The peptides were deprotected and cleaved from the resin in aqueous TFA (95%, 1.5 h). The resin was removed by filtration and the solution was evaporated to dryness in vacuo yielding an oil. The crude S-(Npys)-cysteinylpeptides (see Fig. 4) were dissolved in water, analyzed by HPLC and purified by gel filtration on a column (150 x 1.0 cm) of Sephadex G-25 superfine (eluent: 0.05 M HOAc).

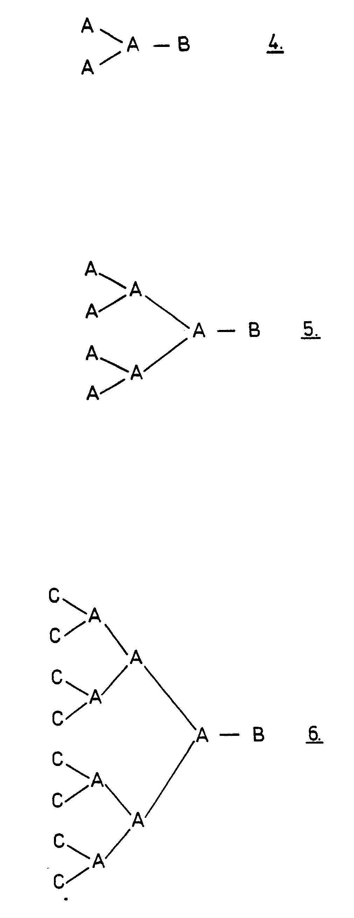

In Fig. 5 the analytical HPLC of crude S(Npys)-cysteinylpeptides (I-III) is shown (detection at 214 nm). Amino acid analysis: I: Asp 1.97 (2), Thr 2.21 (2), Pro 3.62 (4), Ala 2.76 (3), Nle 1.06 (1), Tyr 0.91 (1), His 0.99 (1), Cys n.d. II: Asp 1.91 (2), Thr 0.98 (1), Ser 1.76 (2), Glu 2.12 (2), Pro 2.05 (2), Gly 0.98 (1), Ala 3.96 (4), Val 1.01 (1), Lys 0.88 (1), Cys n.d. III: Asp 1.01 (1), Thr 2.82 (3), Glu 1.00 (1), Pro 1.04 (1), Ile 0.91 (1), Leu 2.18 (2), His 2.98 (3), Cys n.d.

The overall yield of purified S(Npys)-cysteinylpeptides was 40-65% based on the initial loading of the resin.

S-acetyl functionalized BSA

To a stirred solution of 130 mg (2$\mu$mol) BSA in 5.0 mL 0.8M N-ethylmorpholine buffer pH 8.0 a solution of 7.0 mg (40µmol) S-acetylmercaptosuccinic anhydride in 1.5 ml 96% EtOH was added dropwise (10 min). Stirring was continued for another 90 min. The solution was dialyzed against water (2 x 4h, 1 x 16h). The crude material obtained after lyophilization was purified by gel filtration on a Sephadex G-50 fine column (90 x 1.6 cm; eluent: 0.05M HOAc).

To determine the S-acetyl incorporation in the protein 3.75 mg functionalized BSA was dissolved in 4.80 mL 0.1 M sodium phosphate buffer (pH = 8), 0.20 ml of a solution of 100 mg $NH_2OH.HCL$ in 4.00 ml 0.1 M sodium phosphate buffer (pH = 8) was added and the amount of thiol obtained was measured according to Ellman (Arch. Biochem. Biophys. 82, (1959), pages 70-77) using 5,5'-dithio-bis-(2-nitrobenzoic acid). The incorporation turned out to be 14.6 mol S-acetyl groups per mol BSA.


Conjugation of S(Npys)-cysteinylpeptides to S-acetyl functionalized BSA

The S(Npys)-cysteinylpeptides (I-III) were conjugated to the functionalized BSA according to a procedure which is described in detail for peptide I.

An 0.1 M sodium phosphate buffer, purged with nitrogen for 15 min, was used to prepare: 1. a solution of 4.50 mg lyophilized functionalized BSA (S-acetyl loading 14 mol/mol) in 1.00 ml; 2. a solution of 1.15µmol S(Npys)-cysteinylpeptide (I) (based on amino acid analysis) in 1.00 ml; 3. a solution of 100 mg $NH_2OH.HCl$ in 4.00 ml.

To a stirred mixture of solutions 1 and 2, 0.20 mL of solution 3 was added under nitrogen. A strong yellow colour appeared at this stage. After stirring for 3 h the reaction mixture was transferred to a Sephadex G-50 column (90 x 1.6 cm). Elution with 0.05 M HOAc afforded a protein fraction, a peptide fraction and a third, yellow fraction, which contained 3-nitro-2-thiopyridone as the only component detectable at 329 nm (HPLC). Optical density measurements showed that the third fraction contained 0.93µmol 3-nitro-2-thiopyridone ( $\epsilon_{329}$ = 7.3 x $10^3$; 0.05 M HOAc), corresponding to an incorporation of 13.5 mol peptide per mol of protein, assuming that the liberation of 3-nitro-2-thiopyridone is only due to the conjugation of the S(Npys)-cysteinylpeptide to the protein.

Amino acid analysis of the conjugate showed an Asx-Nle ratio of 5.9. The peptide contains two Asn and one Nle, whereas BSA contains no Nle and 54 Asx residues (Adv. Protein Chem. 37, (1985), pages 161-245). This means an incorporation of 13.8 mol peptide per mol protein, which is in good agreement with the result mentioned above.

The S(Npys)-cysteinylpeptide as well as the conjugate were treated with an excess of tri-n-butyl-phosphine in 50% aqueous acetonitrile for 30 min. HPLC analysis (detection at 214 nm) showed that the peptide treatment yielded two main products. One had the same $k'$-value ($k'$ = 1.2) as 3-nitro-2-thiopyridone (Chem. Lett., (1978), 951-952) and was also detectable at 329 nm, whereas the other had the same $k'$-value ($k'$ = 1,7) as the single product detectable in the reaction mixture of the conjugate, evidently the cysteinylpeptide. Amino acid analysis of the peptide produced by treatment of the conjugate with tri-n-butyl-phosphine: Asp 2.12 (2), Thr 1.91 (2), Ala 2.82 (3), Pro 4.00 (4), Nle 1.19 (1), Tyr 0.88 (1), His 1.06 (1), Cys n.d.


Example 5


Kit for use of the conjugation method according to the invention.

A suitable kit contains 5 vials A1, A2, B1, B2 and B3:
- A1: Boc-Cys(Npys)-Opfp
- A2: HOBt (catalyst)
- B1: functionalized carrier protein (e.g. BSA functionalized by reaction with S-acetylmercaptosuccinic anhydride)
- B2: buffer in which the conjugation reaction is to be carried out.
- B3: hydroxylamine.HCl

With A1 and A2 Boc-Cys(Npys) may be coupled to the solid-state peptide (having a free N-terminal group).

After deprotection and removal of the polymer (and optionally purification) the peptide may be conjugated with the carrier protein. For that purpose the S-Npys containing peptide is dissolved in a part of B2. B1 is dissolved in another part of B2. Both parts are mixed. After adding B3 to this mixture and stirring for some time the conjugation is completed. Then the peptide-protein conjugate may be purified by dialysis and/or gelfiltration chromatography.

## Claims

1. A process for preparing an antigenic or immunogenic conjugate such as a multiple antigenic peptide system comprising reaction of a branched structure based on amino acids with an antigenic compound.

2. The process according to Claim 1, wherein the branched structure contains the groups having the formula 4 wherein A is a group derived from an amino acid containing at least one COOH group and two $NH_2$ groups such as lysine and/or ornithine, and B is a group derived from an amino compound such as a diamino compound or an amino acid.

3. The process according to Claim 1 or 2, wherein the branched structure contains the group having the formula 5 wherein A and B are as defined in Claim 2.

4. The process according to Claim 2 or 3, wherein the end groups A are coupled to a solubilizing group C. such as a group derived from polar amino acids, e.g. glutamic acid, as depicted in formula 6.

5. The process according to Claim 4, wherein a spacer group is inserted between groups A and C.

6. The process according to any of the Claims 2-5, wherein the end groups A or C are functionalized in such a way that they contain thiol groups or temporarily protected thiol groups.

7. The process according to Claim 6, wherein said protected thiol groups are represented by the group S-CO-X, X being alkyl, aryl, or arylalkyl.

8. The process according to Claim 6, wherein said protected thiol groups are S-acetyl groups.

9. The process according to any of the preceding claims, wherein the antigenic compound is coupled to a residue containing the 3-nitro-2-pyridine sulfenyl group.

10. The process according to Claim 9, wherein said residue is the S(3-nitro-2-pyridine sulfenyl)cysteinyl group.

11. An antigen or an immunogen on the basis of a conjugate prepared according to the method of any of the preceding claims.

12. An immunogen or an antigen according to Claim 11, which is active against the herpes simples viruses.

13. Use of conjugates obtainable according to any of the claims 1-10 for the preparation of pharmaceutical products.

# Fig -1

1.

```
Ac-S-CH₂-CO-Glu
                  \
                   Lys
Ac-S-CH₂-CO-Glu  /
                       \
                        Lys
Ac-S-CH₂-CO-Glu       /
                  \  /
                   Lys
Ac-S-CH₂-CO-Glu  /
                              \
                               Lys-NH-(CH₂)₃-COOH
Ac-S-CH₂-CO-Glu              /
                  \        /
                   Lys    /
Ac-S-CH₂-CO-Glu  /      /
                     \ /
                      Lys
Ac-S-CH₂-CO-Glu     /
                  \ /
                   Lys
Ac-S-CH₂-CO-Glu  /
```

a. NH₂OH
b. (9-21)-Cys(Npys)   2.

3.

$$[(9-21)-Cys-S-CH_2-CO]_8[Glu]_8[Lys]_7-NH-(CH_2)_3-COOH$$

# Fig-2

fig-3

Fig - 4

I:   H-Cys-Ala-Ala-Thr-Pro-Tyr-His-Pro-Pro-Ala-Thr-Pro-Asn-Asn-Nle-OH
         |
         Npys

II:  H-Cys-Gly-Asp-Asp-Pro-Ser-Pro-Ala-Ala-Lys-Ser-Ala-Val-Thr-Ala-Gln-Glu-OH
         |
         Npys

III: H-Cys-His-Pro-Thr-Thr-Glu-Leu-Asp-Ile-Thr-His-Leu-His-OH
         |
         Npys

Fig - 5

EP 0 339 695 A2

4.

5.

6.